# EUROPÄISCHE PATENTSCHRIFT

(11) **EP 3 138 530 B1**
(45) Veröffentlichungstag und Bekanntmachung des Hinweises auf die Patenterteilung: **02.04.2025**
(21) Anmeldenummer: 16154692.4
(22) Anmeldetag: 08.02.2016
(51) Int. Cl.: A61B 5/107, A61B 1/24, A61C 9/00

(54) **SCANANORDNUNG MIT SCANKOPF**
SCAN ARRANGEMENT WITH SCAN HEAD
ARRANGEMENT DE BALAYAGE AVEC TÊTE DE BALAYAGE

(30) Priorität: 11.08.2015 EP 15180555
(43) Veröffentlichungstag der Anmeldung: 08.03.2017
(73) Patentinhaber: Ivoclar Vivadent AG, 9494 Schaan (LI)
(72) Erfinder: Rohner, Gottfried, 9450 Altstätten (CH); Watzke, Ronny, 6800 Feldkirch (AT); Senti, Theresa, 9497 Triesenberg (LI); Ferilli, Antonio, 9545 Wängi (IT)
(74) Vertreter: Baldus, Oliver

(56) Entgegenhaltungen:
- WO-A1-03/087717
- WO-A1-2010/081498
- WO-A2-2015/013536
- DE-A1- 3 400 781
- US-A1- 2013 003 078
- US-A1- 2013 273 492
- US-A1- 2014 170 587
- US-A1- 2014 276 005
- US-A1- 2014 276 094
- US-A1- 2014 330 133
- US-A1- 2015 209 118
- US-B1- 6 193 658

## Beschreibung

Das technische Gebiet der Erfindung betrifft eine Scananordnung, gemäß dem Oberbegriff von Anspruch 1, sowie ein Verfahren zum Scannen eines Scanbereichs, gemäß dem Oberbegriff von Anspruch 13.

Für das Scannen des Mundinnenraums - oder auch für das Scannen von Mundinnenraumabformungen, also Modellen oder Abdruckmassen, die die Form des Mundinnenraums wiedergeben - ist es bekannt, im Mundinnenraum oder auf dem Modell Referenzpunkte anzubringen. Diese sollen der Orientierung dienen, aber auch die Qualität des sogenannten "Stitching" verbessern, anhand dessen verschiedene Aufnahmebereiche zur Bereitstellung eines dreidimensionalen Gesamtbildes zueinander ausgerichtet werden.

Auch wenn bereits beträchtliche Fortschritte bei den Scanergebnissen zu verzeichnen sind, bestehen nach wie vor gewisse Probleme. So muss der Scankopf dreidimensional geführt werden, was entsprechende Fehlereinflüsse hinsichtlich der Präzision induziert. Auch ist der Kontrast der Scanbereiche, die erfasst werden sollen, recht unterschiedlich. Zur Erfassung kleinerer Erhebungen und Vertiefungen ist vorgeschlagen worden, mit einem schrägen Lichteinfall und der entsprechenden Schattenwirkung zu arbeiten.

Zur Eliminierung von Erkennungsfehlern muss dann aber auch mit Gegenlicht gearbeitet werden, also Lichteinfall aus der Gegenrichtung. Schräger Lichteinfall bedingt jedoch je nach Anordnung des Scanbereiches auch Abdeckungen.

Um diese Nachteile zu verhindern, ist es auch bekannt geworden, mindestens zwei Scanner im Abstand voneinander anzuordnen und auf den gleichen Scanbereich zu richten und so das Höhenprofil stereometrisch zu erfassen. Dies führt jedoch zu erheblichen Kostennachteilen und auch gegebenenfalls räumlichen Problemen gerade in beengten Hohlräumen dem Mundraum.

Diverse optische Untersuchungsvorrichtungen gemäß des Stands der Technik werden zum Beispiel in US2014/0276094A1, DE3400781A1, US2014/0330133A1, WO2010/081498A1 und WO03/087717A1 beschrieben.

In der europäischen Patentanmeldung 15 180 555.3 ist es vorgeschlagen worden, um einen Scanner herum einen Ballon anzuordnen und diesen aufzublasen, so dass er sich an dem Scanbereich anlehnt und an diesem anliegt. Durch die Verformung des Ballons soll die Form des Scannbereichs erkannt werden.

Demgegenüber wäre es jedoch wünschenswert, eine noch flexiblere Lösung zur Verfügung zu stellen.

Um kompliziert geformte Hohlräume scannen zu können, hat man die Scanstrahlung und deren Zuleitung durch den Scankopf zu verbessern versucht. So ist es bereits vorgeschlagen worden, mit unterschiedlichen Frequenzbereichen der elektromagnetischen Strahlungen zu arbeiten. Bei einer feuchten Oberfläche kann es beispielsweise günstig sein, sichtbares Licht oder UV-Licht zu verwenden. Hingegen sind die Reflekionseigenschaften bestimmter Materialien, aus denen der Hohlraum bestehen kann, einerseits bei Ultraschallstrahlung, andererseits aber bei Röntgenstrahlungen besser.

Nachteilig hierbei ist es, dass Scanner mit unterschiedlichen Frequenzbereichen bereitgestellt werden müssen, was die Lösung insgesamt verteuert und teilweise auch unpraktikabel macht.

Daher liegt der Erfindung die Aufgabe zu Grunde, eine Scananordnung gemäß dem Oberbegriff von Anspruch 1 sowie ein Verfahren zum Scannen eines Scanbereichs gemäß dem Oberbegriff von Anspruch 13 zu schaffen, die hinsichtlich des Scanergebnisses ohne nennenswerten Investitionsaufwand deutlich verbessert sind.

Diese Aufgabe wird erfindungsgemäß durch den Gegenstand des Anspruchs 1 bzw. 13 gelöst. Vorteilhafte Weiterbildungen ergeben sich aus den Unteransprüchen.

Besonders günstig ist es, dass sich das folienartige oder flächige, elastisch dehnbare Material an den Scanbereich anlegt. Dies kann durch mechanische Mittel wie geeignet ausgestaltete Rahmenelemente realisiert sein, oder beispielsweise durch Adhäsion zwischen dem Scanbereich und dem Material.

Erfindungsgemäß trägt das Material - oder in modifizierter Ausgestaltung mindestens ein Rahmenelement - ein Muster, das besonders gut scanbar ist. Durch die gute Scanbarkeit ergibt sich ein erheblicher Fortschritt im Bereich mit einem unmittelbaren Scannen des Scanbereichs, der häufig kontrastschwach und in aller Regel ungemustert ist.

Besonders günstig ist es in diesem Zusammenhang, dass sich das Material zum Scankopf hin vorgewölbt erstreckt. Hierdurch lassen sich besonders gut Konturen des entsprechend vorgewölbten Scanbereichs erfassen, und zwar auch dann, wenn der Scanbereich selbst einfarbig und unstrukturiert ist.

Besonders günstig ist es, wenn das Material, das beispielsweise nach der Art einer Membran folienartig oder aber auch netzartig sein kann und eng an dem Scanbereich anliegt, überall die gleiche Dicke aufweist. Diese kann beispielsweise 50 µm oder auch 100 µm betragen. Überraschend wird die Genauigkeit der Erfassung durch die auftragende Dicke des Materials in keiner Weise beeinträchtigt; viel mehr ist sie signifikant höher als bei einem direkten Scan des Scanbereichs.

Die Erfindung ist auch besonders geeignet, erfasste Bilder des Scankopfes über eine dreidimensionale Aneinanderreihung, das sogenannte "Stitching" miteinander zu verbinden. Hierbei lässt sich erfindungsgemäß das Muster in beliebiger geeigneter Weise auswählen, um eine eindeutige örtliche Lokalisierung zu ermöglichen.

Während ein Spannen des Materials zwischen Rahmenelementen, gegebenenfalls gepaart mit oder abgelöst durch Adhäsion, bevorzugt ist, ist es in modifizierter Ausgestaltung der Erfindung vorgesehen, das Material über einen lösbaren Kleber an dem Scanbereich zu fixieren. Diese Lösung hat den Vorteil, dass auch bei Erschütterungen und Bewegungen keine Relativbewegung zwischen dem Scanbereich und dem Material stattfindet.

Demgegenüber hat die vorgespannte Lösung den Vorteil, dass keine Falten entstehen können, bzw. etwaige bestehende Falten gleichsam automatisch weggezogen werden.

Es versteht sich, dass die Ausgestaltung des Musters auf dem Material in weiten Bereichen an die Erfordernisse anpassbar ist. So kann eine Struktur mit Elementen vorgesehen sein, die sehr fein ist und der Auflösung des Scankopf nahe kommt. Besonders günstig ist die Kombination von Musterelementen, die regelmäßig sind, und Musterelementen, die unregelmäßig sind.

Das Muster kann auf der dem Scankopf zugewandten Seite angebracht oder ausgebildet sein, oder aber auch auf der dem Scanbereich zugewandten Seite, wobei es sich in diesem Falle versteht, dass das Material dann für die Scanstrahlung transparent ist.

In einer weiteren bevorzugten Ausgestaltung der Erfindung ist es vorgesehen, die Scanvorrichtung mit Rahmenelementen zu versehen, die sich seitlich neben oder auch auf dem Scanbereich erstrecken und diesem folgen. Wenn der Scanbereich beispielsweise ein Kieferkamm ist, kann das längliche Rahmenelement sich vestibulär und/oder lingual des unbezahnten Kieferkamms erstrecken, oder auf diesem verlaufen. Das Rahmenelement hat dann - in der horizontalen Richtung betrachtet - einen Höhenverlauf, der der okklusalen Fläche des Kieferkamms entspricht, die in dieser Ausführungsform den Scanbereich bildet.

Günstigerweise ist es vorgesehen, dass sich Rahmenelemente des Rahmens entlang des Scanbereichs erstrecken, insbesondere diesem eng benachbart. Sie weisen einen Höhenverlauf auf, der dem Höhenverlauf des Scanbereichs entspricht, wobei die Rahmenelemente mindestens ein zweidimensionales oder dreidimensionales Muster aufweisen. Dieses Muster kann zur Verbesserung des Scanergebnisses auch zusätzlich zum Muster auf dem Material vorgesehen sein.

Eine entsprechende Realisierung ist auch bei einer unbezahnten Prothese möglich.

Das Rahmenelement kann in beliebiger geeigneter Weise ausgewählt werden. Bevorzugt ist es sehr gut plastisch verformbar. Beispielsweise kann ein weiches Metall wie Zinn oder eine Legierung mit Zinn als Kern des Rahmenelements verwendet werden. Diese kann mit einem Schutzüberzug umhüllt sein, der eine Berührung mit dem metallischen Kern des Rahmenelements unterbindet.

Eine weitere Möglichkeit ist die Realisierung des Rahmenelements mit einem Blech, das besonders duktil ist und sich gut verformen lässt.

Das so vorgesehen Blech erstreckt sich dann bevorzugt distal-mesial in seiner Hauptrichtung und mit einer Breite von wenigen Millimetern in lingual-vestibulärer Richtung. Auf diese Weise lässt sich das Blech möglichst genau dem Höhenverlauf des Scanbereichs anpassen und entsprechend verbiegen.

Es kann kurzerhand auf den Scanbereich aufgedrückt und dann gescannt werden.

Auch in diesem Fall ist die Realisierung eines Schutzüberzugs möglich, der die plastische Biegbarkeit des Blechs jedoch nicht beeinträchtigen sollte.

Eine weitere Möglichkeit ist die Realisierung des Kerns des Rahmenelements aus einem plastisch verformbaren organischen Material, beispielsweise aus Bitumen, das widerum mit einem Schutzüberzug umhüllt ist.

Weitere Vorteile, Einzelheiten und Merkmale ergeben sich aus der nachfolgenden Beschreibung mehrerer Ausführungsbeispiele der Erfindung anhand der Zeichnung:
Es zeigen:
- Fig. 1: eine schematische Anordnung einer Scanvorrichtung in teilweiser Schnittansicht;
- Fig. 2: die Anordnung gemäß Fig. 1, jedoch mit einem anders angeordneten Material;
- Fig. 3: die Ausführungsform der Scanvorrichtung gemäß Fig. 1 und Fig. 2, mit einem beispielhaften Muster;
- Fig. 4: eine weitere Ausführungsform des Materials;
- Fig. 5: eine weitere Ausführungsform des Materials, das von Rahmenelementen eines Rahmens gespannt gehalten ist;
- Fig. 6: eine Schnittansicht einer weiteren Ausführungsform des Rahmens gemäß Fig. 5;
- Fig. 7: eine Schnittansicht einer weiteren Ausführungsform des Rahmens gemäß Fig. 6; und
- Fig. 8: eine Schnittansicht einer weiteren Ausführungsform des Rahmens gemäß Fig. 7.

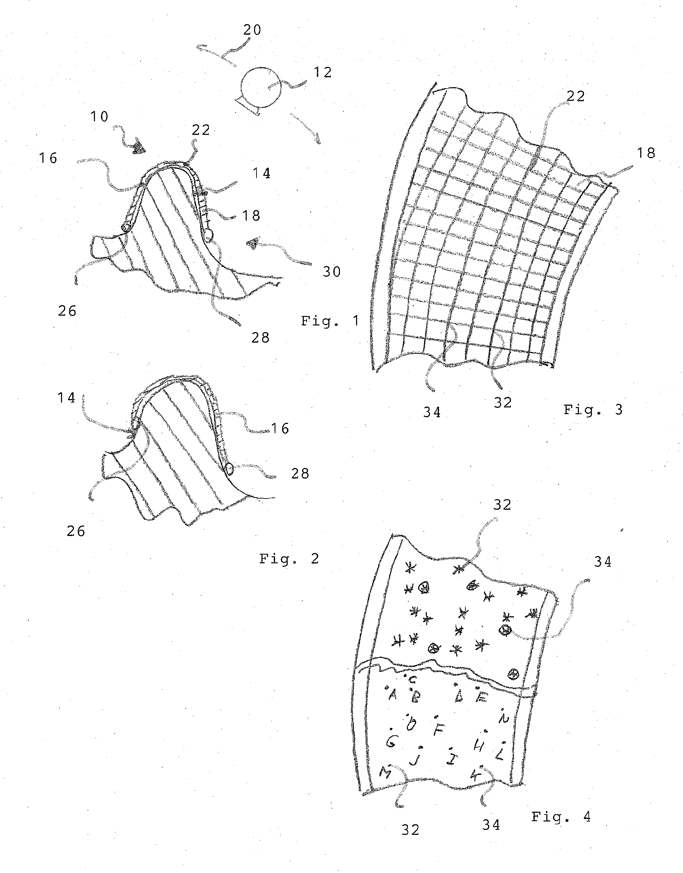

Wie aus Fig. 1 schematisch ersichtlich ist, ist es bei der dargestellten Ausführungsform vorgesehen, dass eine Scananordnung 10 einen Scankopf 12 umfasst, sowie einen Scanbereich 14, der vom Scankopf 12 gescannt werden soll.

Erfindungsgemäß ist der Scanbereich 14 von einem Material 16 abgedeckt, das in dem dargestellten Ausführungsbeispiel eine Membran 18 bildet.

Der Scanbereich 14 ist Teil einer größeren Struktur, die sich dreidimensional erstreckt. Insgesamt kann die Struktur einen Hohlraum bilden, der den Scankopf 12 mindestens teilweise umgibt.

Der in Fig. 1 dargestellte Scanbereich 14 ist in dem dargestellten Ausführungsbeispiel teilweise von dem Material 16 abgedeckt. Für das Scannen kann der Scankopf 12 entlang der Pfeile 20 bewegt werden, aber auch in einer beliebigen anderen dreidimensionalen Richtung, beispielsweise senkrecht zur Zeichnungsebene. Der Scankopf 12 ist auf den Scanbereich 14 und damit auf das Material 16 gerichtet und erfasst dessen Struktur 22.

Wie aus Fig. 1 ersichtlich ist, ist das Material 16 zum Scankopf 12 hin vorgewölbt. Dies liegt in der entsprechenden Form des Scanbereich 14 begründet. Das Material 16 liegt an dem Scanbereich 14 an. Im dargestellten Ausführungsbeispiel ist es zwischen zwei Rahmenelementen 26 und 28 eines Rahmens 30 gespannt gehalten, so dass es eng an dem Scanbereich 14 anliegt.

In dem Zustand gemäß Fig. 1 sind die Rahmenelemente 26 und 28 etwa auf gleicher Höhe.

Das Material 16 kann den gesamten Scanbereich abdecken, also den Bereich, der gescannt werden soll. Es ist aber auch möglich, das Material 16 entlang des Scanbereichs 14 zu verschieben. Dies ist auch Fig. 2 ersichtlich; aus dem Vergleich der Figuren 1 und 2 ergibt sich, dass die Erstreckung des Materials 16 gleich lang ist, jedoch das Rahmenelement 26 gemäß Fig. 2 höher angeordnet ist als das Rahmenelement 28 in dieser Figur.

Das Material 16 ist in dem dargestellten Ausführungsbeispiel als dünne Membran ausgebildet. Die Wandstärke der Membran beträgt beispielsweise 200 µm. Durch die Spannung zwischen den Rahmenelementen 26 und 28 ist die Wandstärke weiter reduziert, beispielsweise auf 150 µm.

Die Struktur 22 der Membran 18 ist so ausgewählt, dass sie von dem Scankopf 12 besonders gut erkennbar ist. Ein Beispiel für eine derartige Struktur 22 ist aus Fig. 3 ersichtlich. Die Struktur besteht aus einer Vielzahl von Musterelementen 32, 34, die voneinander regelmäßig beabstandet sind, aber bevorzugt auch aus Musterelementen, die unregelmäßig zueinander angeordnet sind (Fig. 4).

Durch die Verformung der Membran 18 ergibt sich eine entsprechend der Dehnung der Membran geänderte Anordnung der Musterelemente 34 und 32 zueinander, aus welcher sich die Form des Scanbereichs 14 bestimmen lässt.

Besonders günstig lässt sich dies realisieren, wenn regelmäßig zueinander angeordnete und unregelmäßig zueinander angeordnete Musterelemente 32 und 34 miteinander kombiniert sind. Ein Beispiel für derartige unregelmäßig angeordnete Musterelemente ist aus Fig. 4 ersichtlich.

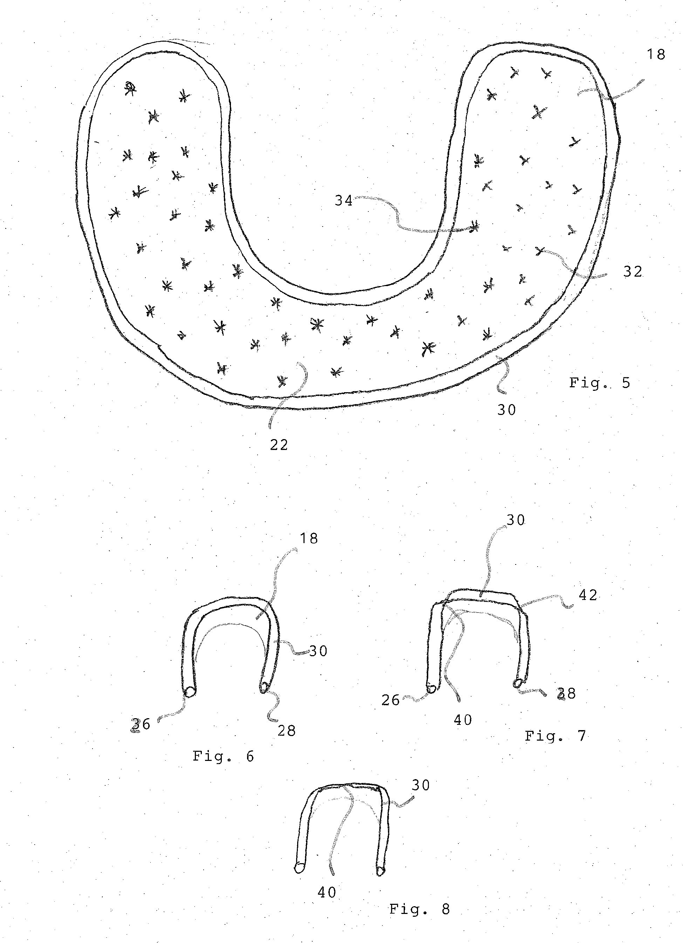

Aus Fig. 5 ist eine modifizierte Version der Membran 18 ersichtlich. Bei dieser Version ist der dortige Rahmen 30 umlaufend ausgebildet und spannt die Membran 18 auf.

Der Rahmen 30 und damit die Membran 30 ist dreidimensional verformbar. In der Draufsicht bildet die Form des Rahmens gemäß Fig. 5 ein U, und gemäß den Figuren 6 bis 8 ist der Rahmen 30 so verformbar, dass in der Seitenansicht im Schnitt sich ebenfalls ein U realisieren lässt.

Eine Anwendung wäre beispielsweise das Scannen einer bereits erzeugten Prothese, um deren Oberfläche festzustellen, oder aber das Scannen eines Kiefers im Mund eines Patienten.

Um die Spannung in der Membran aufrecht zu erhalten und zu erreichen, dass auch kleinste Unebenheiten des Scanbereichs erfasst werden, ist es vorgesehen, die in Fig. 6 dargestellte Anordnung aus dem Rahmen 30 und der nicht ersichtlichen und in dem Rahmen gespannten Membran 18 anzudrücken oder in beliebiger anderer Weise dafür zu sorgen, dass der Kontakt zwischen dem Scanbereich und der Membran 18 sicher aufrechterhalten bleibt.

Um das erwünschte doppelte Abkippen des Rahmens 30 sicherzustellen, kann gemäß Fig. 6 die Realisierung von Filmscharnieren 40 und 42 zwischen dem Basisschenkel und den Seitenschenkeln des U vorgesehen sein. Diese lassen sich durch Verdünnung des Materials, aus dem der Rahmen 30 besteht, leicht realisieren.

Gemäß Fig. 8 ist lediglich ein Filmscharnier 40 vorgesehen, das sich mittig - etwa in der Basis des U - erstreckt und die erwünschte Vorformbarkeit gewährleistet, während die dem Filmscharnier 40 benachbarten Bereiche des Rahmens 30 dünn ausgebildet sind und daher flexibel sind.

Es versteht sich, dass die Erfindung nicht auf die dargestellten Ausgestaltungen beschränkt ist. Beispielsweise kann anstelle der Struktur 22 eine beliebige andere Struktur mit anderen Musterelementen 32, 34 realisiert sein. Es ist auch möglich, das Reflektions- bzw. Absorbtionsmaximum der Musterelemente 32 und 34 an den erfassten Wellenlängenbereich des Scankopfs 12 anzupassen. Ferner kann anstelle des Rahmens 30 ein lösbarer Kleber verwendet werden, der die Membran 18 auf dem Scanbereich 14 hält. Dies kann auch kurzerhand durch Adhäsion erfolgen.

Anstelle der folienartigen Membran kann in modifizierter Ausgestaltung auch ein Netz oder eine sonstige Ausnehmungen aufweisende Struktur, die Musterelemente aufweist, verwendet werden. Auch diese kann ie nach Anwendungsfall über einen Rahmen 30. über einen Kleber oder über Adhäsion an dem Scanbereich 14 anliegend gehalten sein.

Der Schutzumfang der Erfindung ist in den anhängenden Ansprüchen definiert.

## Patentansprüche

1. Scananordnung zum scannen einer dreidimensionalen räumlichen Struktur, die einen Scanbereich bildet, mit einem Scankopf und einem flächigen, oder einem folienartigen, eine Membran (18) bildenden, Material (16), das eingerichtet ist den Scanbereich (14) mindestens teilweise so abzudecken, dass das Material (16) flächig, an dem Scanbereich anliegt,
**dadurch gekennzeichnet, dass** das Material (16) durch Spannung zwischen plastisch verformbaren Rahmenelementen (26, 28) eines Rahmens (30) so gehalten ist, dass das Material (16) sich zum Scankopf (12) vorgewölbt erstreckt.

2. Scananordnung nach Anspruch 1, **dadurch gekennzeichnet, dass** das Material (16) elastisch dehnbar ist und der dreidimensionalen Oberflächenform des Scanbereichs (14) bei Anlage an diesem folgt.

3. Scananordnung nach Anspruch 2, **dadurch gekennzeichnet, dass** das elastisch dehnbare Material (16) an dem Scanbereich (14) über den gesamten Scanbereich (14) hinweg, in mindestens einer Richtung unter einer vorgegebenen Vorspannung vorgespannt, anliegt.

4. Scananordnung nach einem der vorhergehenden Ansprüche, **dadurch gekennzeichnet, dass** das elastisch dehnbare Material (16) zwischen zwei Rahmenelementen (26, 28) gehalten ist, zwischen denen es vorgespannt ist, wobei die Vorspannung insbesondere eine Materialdehnung zwischen 1 Prozent und 20 Prozent in Richtung zwischen den Rahmenelementen (26, 28) auslöst, und dass das Material (16) zwischen den Rahmenelementen (26, 28) gepaart mit Adhäsion gehalten ist.

5. Scananordnung nach einem der vorhergehenden Ansprüche, **dadurch gekennzeichnet, dass** das elastisch dehnbare Material (16), aus Sicht des Scanbereichs (14) betrachtet, sich konkav um diesen herum erstreckt.

6. Scananordnung nach einem der vorhergehenden Ansprüche, **dadurch gekennzeichnet, dass** die Membran (18) und/oder mindestens ein Rahmenelement (26,28) ein zweidimensionales oder dreidimensionales Muster aufweist, das sich im Wesentlichen über die gesamte Erstreckung der Membran (18) und damit über den Scanbereich (14) erstreckt, wobei das Muster insbesondere eine Struktur (22) mit Elementen aufweist, deren Abstand höchstens das 10-fache der Auflösung des Scankopfes (12) beträgt.

7. Scananordnung nach Anspruch 6, **dadurch gekennzeichnet, dass** das Muster eine dreidimensionale Struktur (22) mit Musterelementen (32, 34) aufweist, die mindestens teilweise eine Regelmäßigkeit und/oder periodische Abfolgen aufweist.

8. Scananordnung nach Anspruch 6 oder Anspruch 7, **dadurch gekennzeichnet, dass** das Muster eine zwei- oder dreidimensionale Struktur (22) aufweist, deren Musterelemente (32, 34) unregelmäßig angeordnet sind.

9. Scananordnung nach einem der vorhergehenden Ansprüche, **dadurch gekennzeichnet, dass** die Membran (18) eine Wandstärke von weniger als 0,5 mm, insbesondere von etwa 200 µm in unverformten Zustand aufweist, und mit dem Muster auf einer Seite, insbesondere der dem Scankopf (12) zugewandten Seite, aufgebracht ist.

10. Scananordnung nach einem der vorhergehenden Ansprüche, **dadurch gekennzeichnet, dass** der Scankopf (12) an eine Auswerteinrichtung mit einer Bildverarbeitungsvorrichtung angeschlossen ist, die insbesondere per Stitching aneinander angrenzende Bilder der verformten Membran (18) zu einem Gesamtbild zusammenfügt.

11. Scananordnung nach einem der vorhergehenden Ansprüche, **dadurch gekennzeichnet, dass** die Rahmenelemente (26, 28) zur Bildung eines Rahmens (30) an Stirnseiten der Membran (18) miteinander verbunden sind, insbesondere, im Querschnitt betrachtet, in U-Form vorgespannt.

12. Scananordnung nach Anspruch 11, **dadurch gekennzeichnet, dass** die Rahmenelemente (26, 28) an den Stirnseiten der Membran (18) gelenkig miteinander verbunden sind.

13. Verfahren zum Scannen eines Scanbereichs mit einer Scananordnung nach einem der vorhergehenden Ansprüche, wobei der Scankopf (12) geführt ist, um den Scanbereich (14) abzuscannen, wobei der Scanbereich (14) von der Membran (18), welche an dem Scanbereich anliegend durch Spannung zwischen plastisch verformbaren Rahmenelementen (26, 28) des Rahmens (30) gehalten wird, abgedeckt ist, und wobei der Scankopf (12) an eine Auswerteinrichtung angeschlossen ist, die, basierend auf der Verformung der Membran (18), die über ein auf dieser aufgebrachtes Muster erkannt wird, die Form des Scanbereichs (14) erfasst.

## Claims

1. A scanning arrangement for scanning a three-dimensional spatial structure which forms a scanning area, comprising a scan head and a flat or a film-like material (16) forming a membrane (18), which is configured to cover the scanning area (14) at least partially in such a way that the material (16) is in flat contact with the scanning area, **characterized in that** the material (16) is held by tension between plastically deformable frame elements (26, 28) of a frame (30) in such a way that the material (16) extends towards the scan head (12) in a bulged manner.

2. The scanning arrangement according to claim 1, **characterized in that** the material (16) is elastically stretchable and follows the three-dimensional surface shape of the scanning area.
(14) when it is in contact therewith.

3. The scanning arrangement according to claim 2, **characterized in that** the elastically stretchable material (16) is in contact with the scanning area (14) over the entire scanning area (14), prestressed in at least one direction under a predetermined prestress.

4. The scanning arrangement according to one of the preceding claims, **characterized in that** the elastically stretchable material (16) is held between two frame elements (26, 28), between which it is prestressed, the prestress in particular triggering a material stretch of between 1 percent and 20 percent in the direction between the frame elements (26, 28), and **in that** the material (16) is held between the frame elements (26, 28) paired with adhesion.

5. The scanning arrangement according to one of the preceding claims, **characterized in that** the elastically stretchable material (16), as seen from the scanning area (14), extends concavely around it.

6. The scanning arrangement according to one of the preceding claims, **characterized in that** the membrane (18) and/or at least one frame element (26,28) has a two-dimensional or three-dimensional pattern which extends substantially over the entire extent of the membrane (18) and thus over the scanning area (14), wherein the pattern has in particular a structure (22) with elements whose spacing is at most 10 times the resolution of the scan head (12).

7. The scanning arrangement according to claim 6, **characterized in that** the pattern has a three-dimensional structure (22) with pattern elements (32, 34), which has at least partially a regularity and/or periodic sequences.

8. The scanning arrangement according to claim 6 or claim 7, **characterized in that** the pattern has a two- or three-dimensional structure (22) whose pattern elements (32, 34) are arranged irregularly.

9. The scanning arrangement according to one of the preceding claims, **characterized in that** the membrane (18) has a wall thickness of less than 0.5 mm, in particular of about 200 µm in an undeformed state, and is applied with the pattern on one side, in particular the side facing the scan head (12).

10. The scanning arrangement according to one of the preceding claims, **characterized in that** the scan head (12) is connected to an evaluation device with an image processing device which, in particular by stitching, combines adjacent images of the deformed membrane (18) to form an overall image.

11. The scanning arrangement according to one of the preceding claims, **characterized in that** the frame elements (26, 28) are connected to one another to form a frame (30) at the end faces of the membrane (18), in particular, when viewed in cross-section, prestressed in a U-shape.

12. The scanning arrangement according to claim 11, **characterized in that** the frame elements (26, 28) are hingedly connected to one another at the end faces of the membrane (18).

13. A method of scanning a scanning area with a scanning arrangement according to one of the preceding claims, wherein the scan head (12) is guided to scan the scanning area (14), wherein the scanning area (14) is covered by the membrane (18), which is held in contact with the scanning area by tension between plastically deformable frame elements (26, 28) of the frame (30), and wherein the scan head (12) is connected to an evaluation device which detects the shape of the scanning area (14) based on the deformation of the membrane (18), which is recognized via a pattern applied thereto.

## Revendications

1. Dispositif de balayage pour balayer une structure spatiale tridimensionnelle qui forme une zone de balayage, avec une tête de balayage et un matériau (16) plat ou de type feuille formant une membrane (18), qui est conçu pour couvrir la zone de balayage (14) au moins partiellement de telle manière que le matériau (16) est appliqué à plat contre la zone de balayage, **caractérisé en ce que** le matériau (16) est maintenu par tension entre des éléments de cadre plastiquement déformables (26, 28) d'un cadre (30) de telle manière que le matériau (16) s'étend de manière bombée vers la tête de balayage (12).

2. Dispositif de balayage selon la revendication 1, **caractérisé en ce que** le matériau (16) est élastiquement extensible et suit la forme de surface tridimensionnelle de la zone de balayage (14) lorsqu'il est en contact avec celle-ci.

3. Dispositif de balayage selon la revendication 2, **caractérisé en ce que** le matériau élastiquement extensible (16) adhère à la zone de balayage (14) sur toute la zone de balayage (14), précontraint dans au moins une direction sous une tension initiale prédéterminée.

4. Ensemble de balayage selon l'une des revendications précédentes, **caractérisé en ce que** le matériau élastiquement extensible (16) est maintenu entre deux éléments de cadre (26, 28) entre lesquels il est précontraint, la précontrainte induisant notamment un allongement du matériau compris entre 1 pour cent et 20 pour cent dans la direction entre les éléments de cadre (26, 28), et **en ce que** le matériau (16) est maintenu entre les éléments de cadre (26, 28) combiné avec de l'adhérence.

5. Dispositif de balayage selon l'une des revendications précédentes, **caractérisé en ce que** le matériau élastiquement extensible (16) s'étend de manière concave autour de la zone de balayage (14) lorsqu'il est considéré du point de vue de celle-ci.

6. Dispositif de balayage selon l'une des revendications précédentes, **caractérisé en ce que** la membrane (18) et/ou au moins un élément de cadre (26, 28) présente un motif bidimensionnel ou tridimensionnel s'étendant substantiellement sur toute l'extension de la membrane (18) et donc au-dessus de la zone de balayage (14), où le motif présente en particulier une structure (22) avec des éléments dont la distance est au maximum égale à 10 fois la résolution de la tête de balayage (12).

7. Dispositif de balayage selon la revendication 6, **caractérisé en ce que** le motif présente une structure tridimensionnelle (22) avec des éléments de motif (32, 34) qui présente une régularité au moins partielle et/ou des séquences périodiques.

8. Dispositif de balayage selon la revendication 6 ou la revendication 7, **caractérisé en ce que** le motif présente une structure bidimensionnelle ou tridimensionnelle (22) dont les éléments du motif (32, 34) sont disposés de manière irrégulière.

9. Dispositif de balayage selon l'une des revendications précédentes, **caractérisé en ce que** la membrane (18) présente une épaisseur de paroi inférieure à 0,5 mm, en particulier environ 200 µm à l'état non déformé, et est appliquée avec le motif sur une face, en particulier sur la face tournée vers la tête de balayage (12).

10. Dispositif de balayage selon l'une des revendications précédentes, **caractérisé en ce que** la tête de balayage (12) est reliée à un dispositif d'évaluation comportant un dispositif de traitement d'images qui assemble en particulier des images adjacentes de la membrane déformée (18) pour former une image globale.

11. Dispositif de balayage selon l'une des revendications précédentes, **caractérisé en ce que** les éléments de cadre (26, 28) sont reliés entre eux pour former un cadre (30) sur les côtés frontaux de la membrane (18), en particulier, lorsqu'ils sont vus en coupe, précontraints en forme de U.

12. Dispositif de balayage selon la revendication 11,
**caractérisé en ce que** les éléments du cadre (26, 28) sont reliés entre eux de manière articulée au niveau des faces frontales de la membrane (18).

13. Procédé de balayage d'une zone de balayage avec un dispositif de balayage selon l'une des revendications précédentes, où la tête de balayage (12) est guidée pour balayer la zone de balayage (14), où la zone de balayage (14) est recouverte par la 2e membrane (18), qui est maintenue en place contre la zone de balayage par une tension entre des éléments de cadre (26, 28) déformables en plastique du cadre (30), et où la tête de balayage (12) est reliée à un dispositif d'évaluation, qui détecte la forme de la zone de balayage (14) en se basant sur la déformation de la membrane (18) qui est détectée par un motif appliqué sur celle-ci.
